# EUROPEAN PATENT SPECIFICATION

(11) **EP 0 530 322 B1**
(45) Date of publication and mention of the grant of the patent: **25.09.1996**
(21) Application number: 91912058.4
(22) Date of filing: 14.05.1991
(51) Int. Cl.: C12Q 1/02, C12Q 1/20, C12Q 1/04, C12Q 1/10, C12Q 1/34, C12N 1/38

(54) **NOVEL AND IMPROVED METHOD FOR DETERMINATION OF E. COLI IN WATER**
NEUES UND VERBESSERTES VERFAHREN ZUR BESTIMMUNG VON E. COLI IN WASSER
PROCEDES NOUVEAUX ET AMELIORES DE DETERMINATION DE E. COLI DANS L'EAU

(30) Priority: 14.05.1990 US 523320
(43) Date of publication of application: 10.03.1993
(73) Proprietor: THE REGENTS OF THE UNIVERSITY OF CALIFORNIA, Oakland, California 94612-3550 (US)
(72) Inventor: CHANG, George, W., Berkeley, CA 94703 (US); LUM, Rosalind, A., Saratoga, CA 95070 (US)
(74) Representative: Goldin, Douglas Michael
(86) International application number: PCT/US91/03404
(87) International publication number: WO 91/18111

(56) References cited:
- US-A- 3 870 601
- US-A- 4 923 804
- US-A- 4 925 789
- JOURNAL OF FOOD PROTECTION vol. 53, no. 10 , 1 July 1990 , WASHINGTON DC USA page 910 G.W. CHANG ET AL. 'Tactics for combining the coliform and indole tests: simple media for both total coliforms and Escherichia coli.'
- ABSTRACTS OF THE GENERAL MEETING OF THE AMERICAN SOCIETY OF MICROBIOLOGY vol. 91 , 1991 , WASHINGTON DC USA page 316 G.W. CHANG ET AL. 'Colitag: an indole- and MUG-based medium for detecting and recognizing fecal coliforms and Escherichia coli.'
- JOURNAL OF CLINICAL MICROBIOLOGY, vol. 18, no. 6, December 1983; S.C. EDBERG et al., pp. 1287-1291/
- JOURNAL OF CLINICAL MICROBIOLOGY, vol. 24, no. 3, September 1986; S.C. EDBERG et al., pp. 368-371/
- JOURNAL OF CLINICAL MICROBIOLOGY, vol. 19, no. 2, February 1984; R.W. TREPETA et al., pp. 172-174/
- E. JAWETZ et al., "Review of Medical Microbiology", 1987, Appleton & Lang (Publ.), Norwalk, CT (US); p. 233/
- B.D. DAVIS et al., "Microbiology", 1980, Harper & Row (Publ.), New York, NY (US); table 31-4/
- DIFCO LABORATORIES, "Difco Manual", 1984; Difco Laboratories (Publ.), Detroit, MI (US); "Bacto Indole Test Strips"/
- APPLIED & ENVIRONMENTAL MICROBIOLOGY, vol. 54, no. 8, August 1988; J.P. BERG et al., pp. 2118-2122/
- APPLIED & ENVIRONMENTAL MICROBIOLOGY, vol. 54, no. 6, June 1988; S.C. EDBERG et al., pp. 1595-1601/

## Description

### Field of the Invention

This invention concerns a novel, simple, rapid, sensitive, specific and inexpensive method for determination of the level of total coliform and E. coli in water samples. The method is based on two definite characteristic traits of E. coli, namely on lactose utilization and on indole formation.

### Background and Related Disclosures

Escherichia coli (E. coli) is a gram-negative facultatively anaerobic bacteria normally inhabiting the gastrointestinal tract which is found in human and animal feces. E. coli is a frequent cause of infections of the urogenital tract and diarrhea.

Since E. coli is normally found in feces, its presence in water has been long recognized and used as an indicator of possible fecal contamination of water and food. Therefore, much effort has been expended in devising and improving methods for detection, identification and quantitation of these bacteria.

However, E. coli have a major shortcoming as water contamination indicator bacteria: there has been no simple and specific way to detect them. Therefore, people have simply detected coliform bacteria, a broader subset of Enterobacteriaceae, which includes environmental organisms as well as fecal E. coli, but excludes Salmonella, Shigella and Proteus. Consequently, coliform bacteria became a measure of drinking water contamination by fecal matter.

Currently available tests for determination of the presence of coliform and in particular E. coli are based on results of characterization tests which place the unknown organism in a defined group. Most commonly used tests are enzymatic tests where the presence of an enzyme known to be involved in the bacteria metabolism is detected subjectively by observation of a change in the appearance, a change in the color, a change in the fluorescence or some other chemical indication. Methods in Microbiology, 19:105 (1987).

Most existing tests for coliform and for E. coli are based on the fact that Escherichia ferments glucose and lactose and as a result of such fermentation, gas or acid is produced. Forsch. Med, 3:515 (1885). A compendium of methods, currently available and used for examination of water contamination, is published by American Public Health Association, (1985). Most of the currently used methods utilize enzymes involved in the metabolism of lactose, such as the coliform bacterial enzyme β-D-galactosidase which catalyzes the first step of the metabolic pathway which converts lactose to acid and gas.

Research early in the 20th century led to a classical fecal coliform test involving incubation of cultures at 44.5°C. At this temperature, E. coli grows and produces gas or acid from lactose, but environmental coliforms will not.

Two primary disadvantages are associated with the fecal coliform technique. First, the procedure is lengthy. Due to the rather high temperatures of 44°C which must be used for bacterial incubation, the procedure is often lethal for stressed or injured bacteria. Therefore, it is customary to inoculate fecal coliform tubes with fully grown cultures from a presumptive coliform test. Such procedure takes two days. Alternatively, fecal coliform tubes may be incubated for a short while at 35°C followed by 44.5°c incubation. However, the gain in the shortening of the test time is outweighed by additional manipulation involving timing of two incubations and additional handling. Second, the procedure has a limited specificity because it recognizes as undesirable and harmful E. coli also around 10% of coliforms which are not E. coli. Appl. Environ. Microb., 55:335 (1989). Third, the procedure is performed in culture tubes, each equipped with an inverted second tube (Durham tube), where the gas bubbles accumulate and may be observed.

In the clinical laboratory, many attempts to develop better tests for screening of fully grown pure colonies of pathogenic members of the family Enterobacteriaceae were made. In 1984, J. Clin. Microbiol., 20:136, described a single tube, multiple test medium for identification of fully grown pure cultures of Enterobacteriaceae from enteric and other clinical specimens. The used medium allows detection of motility, β-galactosidase, phenylalanine deaminase activity and hydrogen sulfide and indole production. However, the test can be done only on fully grown pure colonies and is thus not useful for water samples.

Lately, the presence of β-glucuronidase (GUR) became a recognized indicator of the presence of E.coli, in the hospital. In 1976, Acta Pathol. Microbiol. Scand. Sect.B, 84:245 (1976) described findings showing that 97% of clinical isolates of E. coli produce β-D-glucuronidase (GUR), whereas most other coliform bacteria do not. The success of GUR on clinical samples motivated scientists to apply it for E. coli detection in food and water.

One very serious flaw of the β-glucuronidase procedure is that it presumes that all or almost all E. coli are able to produce β-D-glucuronidase. Based on this presumption, a number of techniques, including the Rapid Identification Method (RIM) for E. coli were developed. The RIM technique, referenced below, involves simultaneous measurements of β-glucuronidase and β-galactosidase, followed by determination of the presence of indole.

A conventional test for identifying fully grown pure culture of E. coli consists of adding O-Nitrophenyl-β-D-galactopyranoside (ONPG) in Sorensen phosphate buffer having pH 7.5 to a fully grown test isolate and incubating the mixture for 1 hour at 35°C. The presence or absence of E. coli is determined by presence or absence of a yellow color which indicates the presence of β-galactosidase. If there is no color change in the original colorless solution, results are read as negative, and the sample is presumed not to contain E. coli. The conventional test is described in J. Clin. Mirobiol., 18:1287 (1983). Two subsequently developed tests are RIM and Rapid Detect E. coli (RDE). Both are based on determination of β-glucuronidase.

The RIM system consists of reagents-impregnated cotton swabs on wooden sticks with which fully grown bacterial colonies are touched and the swab containing the bacterial inoculum is placed in specific RIM buffer containing ONPG, incubated at 35°C for 30-60 minutes. The positive ONPG reaction is noted with the appearance of the yellow color. If positive, the presence of β-glucuronidase activity is subsequently determined by detecting fluorescence upon irradiation with 366 nm UV light. If the β-glucuronidase test is positive, Kovacs reagent is added for determination of indole presence indicated by red color.

The RDE test is somehow improved system for determination of β-glucuronidase which utilizes a paper disk impregnated with substrates. In RDE, fully grown bacterial colonies are inoculated into distilled water to yield a bacterial suspension. Then, a disk containing both β-glucuronidase and ONPG substrates are added to the tube which is incubated at 35°C. Development of yellow color indicates the presence of β-galactosidase, which hydrolyzes the synthetic substrate ONPG. Hydrolysis of ONPG indicates the presence of β-galactosidase and thus the presence of lactose-fermenting organisms like Escherichia A-D group, Citrobacter and Arizona. When the β-galactoside test is positive, the presence of β-glucuronidase is determined, as in the RIM test, by the examination of the tube with a 366 nm UV light source. If there is a fluorescence, there is β-glucuronidase which has been found to be present in about 97% of all clinical isolates of E. coli. Acta Pathol. Microbiol. Scand. Sect. B, 84:245 (1976). If the β-glucuronidase test is negative, an indole indicator test is added to the sample to test for indole production. A red color indicates the presence of indole. Only when all three tests, i.e., ONPG, β-glucuronidase and indole are positive, it is concluded that an organism is presumably E. coli. If any of the three tests is negative, the test is interpreted as denoting an organism having a low probability of being E. coli. In such an event, alternative procedures for confirmatory identification of clinical isolates are performed. J. Clin. Microbiol., 24:368 (1986).

The earlier observation that about 97% of all clinical E. coli produce β-glucuronidase, led to the assumption that 97% of all E. coli from any source produce the enzyme. Recently, however, it has been found that such presumption was false and that around 30-50% of E. coli may be β-glucuronidase negative.

Notwithstanding, the 97% glucuronidase-positive E. coli premise led consequently to a development of so called MMO-MUG (Minimal Medium ONPG-methylumbelliferyl-β-D-glucuronidase) test currently approved by the US EPA as one of the analytical methods to enumerate total coliform.

The MMO-MUG test uses a fluorescent compound MUG as a β-glucuronidase substrate. The test is based on introducing a tested sample in the MMO-MUG medium and on observation of production of β-galactoside from ONPG, as evidenced by the formation of a yellow color in 24 hours, followed by formation of a fluorescent substance from 4-methylumbelliferyl-β-D-glucuronide, a substrate for β-glucuronidase. The MMO-MUG test consists of specific complex medium consisting of various salts in combination with amphotericin B, ONPG, MUG, and solanium. The MMO-MUG test is approved for estimating of total coliform bacteria and is not specific enough to distinguish E. coli from other coliforms. Timewise, the test requires the incubation for at least 24 hours. Most importantly, the MMO-MUG test has a false positive rate between 13-35%, which error, for lack of any better test available, was found to be acceptable by the US EPA.

Most serious flaw of this test, however, is that only about 66-70% of all E. coli from fecal samples are β-glucuronidase-positive and about 30-34% of E. coli is β-glucuronidase-negative. Consequently, in about 30-34% the MMO-MUG test gives false negative results. Appl. Environ. Microbiol., 55:335 (1989) shows that about 30-34% of all fecal isolates of E. coli are β-D-glucuronidase-negative when measured with lauryl sulfate tryptose broth containing MUG and among those β-glucuronidase-positive there is certain number of β-glucuronidase-positive bacteria which were shown to be temperature dependent for β-glucuronidase production. In these temperature dependent E. coli, such production was very weak at 37°C but strongly positive at 44.5°C. Moreover, some fecal samples contained only β-glucuronidase-negative E.coli. Since the samples analyzed in the above study came from the fecal samples of healthy subjects, it is clear that the fecal contamination of water by E. coli may not be detected in as many as 30-35% when the tests described above are used. This false-negative rate has serious implications for public health. When samples of water from the Southern California, U.S.A. were examined, about 40% of the E. coli were found to be negative in MMO-MUG (M. Stewart and B. Olson, Abstracts: Ann. Meeting Amer. Soc. Microbiol., (1990).

The high false negative rate was also confirmed in other studies. A collection of fecal E. coli from many parts of the world was examined with both LT-MUG and the MMO-MUG technique, and a 30% false-negative rate was found J. Food Prot. 55:972 (1990).

The indole-producing enzyme tryptophanase was one of the first enzyme tests used for identification of E. coli. In the 100 years since Kitasato described the indole test in Zeitschrift Hyg. Leipzig, 7:515 (1889), it has proven to be one of the most useful and definitive test for E. coli. It is used, as described in classical IMViC test published in Identification of Enterobacteriaceae, 2nd Ed., Burgess Publ. (1962), for distinguishing E. coli from other coliforms.

The major disadvantages of the indole test is that it cannot be combined with tests for lactose utilization and used as a simple, one-tube test for E. coli in water samples.

The principle of the indole test is the ability of E. coli to form tryptophanase, the enzyme which metabolizes tryptophane into indole, which ability seems to be closely related to ability of E. coli to grow in mammals intestinal tract. The formation of tryptophanase is inducible by tryptophan and its analogues. Since a number of species of Enterobacteriaceae has ability to produce tryptophanase, the presence of this enzyme results in the accumulation of indole as a catabolite of tryptophan and thus the presence of this enzyme is very useful for identification of Enterobacteriaceae pathogens.

The standard medium used for the identification of indole production, described in Standard Methods for Water and Wastewater Analysis requires incubation at 35°C for 24 hours in a carbohydrate-free medium and testing for indole with Kovacs reagent (Zeit. Immunitaetforsch. Exp. Ther., 55:311 (1928)). This reaction is based on colorimetric reaction of indole with p-dimethylaminobenzaldehyde. In this test, free indole is removed by extraction to liquid media such as amyl alcohol or xylene to distinguish free indole from residual tryptophan, which also reacts with p-dimethylaminobenzaldehyde under suitable conditions.

An alternative colorimetric reaction for indole described in J. Appl. Bacteriol., 63:329 (1987) is based on condensation of indole with free α-or β-positions in the pyrrole ring, in acid solution with glutaconic aldehyde to form red-violet polymethine dyes. A microtest, based on a similar idea, which is useful for rapid identification of Enterobacteriaceae is described in Acta Path. Microbiol. Immunol. Scand. Sect. B, 92:239 (1984).

USP 3,870,601 provides a culture medium suitable for distinguishing between bacteria of the family Enterobacteriaceae, which comprises a chromogenic β-galactoside and another substrate.

The deficiencies and disadvantages connected with the methods, tests and techniques currently available which are described above, are overcome with the method of the current invention.

### SUMMARY

One aspect of this invention is a sensitive, fast, specific, accurate and inexpensive method for determination of the presence of total coliform and E.coli in water samples.

The method can be used for quantitative determination of a most probable number of E.coli present in the water sample.

Another aspect of this invention are testing methods for quick visual determination of the presence of E.coli and coliform bacteria in water (ECOTAG), for determination of total coliform and E.coli in water samples (COLITAG) and for enumeration of the number of coliforms and E.coli present (ECOTAG-QUANTI).

Accordingly, the invention provides a method for specifically detecting coliform bacteria and *E.coli* in a water sample, which method comprises steps:
(a) contacting said sample with a medium comprising:
   (i) a lactose surrogate which is a β-D-galactosidase substrate;
   (ii) tryptophan or a tryptophan-substitute which is a tryptophanase substrate;
   (ii) a protein or peptide hydrolysate in an amount sufficient to promote the growth of *E.coli;*
(b) incubating said bacteria at a temperature of 44.5°C for 2 to 24 hours; then
(c) determining the presence or absence of a β-D-galactosidase reaction product of said β-D-galactosidase substrate;
(d) determining the presence or absence of a tryptophanase reaction product of said tryptophanase substrate;
wherein the presence of said β-D-galactosidase reaction product indicates the presence of a coliform bacterium in said sample and the presence of said tryptophanase reaction product indicates the presence of *E.coli* in said sample.

In a preferred embodiment, the invention provides a method for specifically detecting coliform bacteria and *E.coli* in a water or food sample, which method comprises steps:
(a) forming an aqueous sample medium mixture by contacting said sample with a medium comprising:
   (i) an indole-producing tryptophanase substrate;
   (ii) a chromogenic or fluorogenic β-D-galactosidase substrate;
   (iii) a protein or peptide hydrolysate in an amount sufficient to promote the growth of *E.coli* in said mixture;
(b) incubating said mixture at a temperature of 44.5°C for 2 to 24 hours; thereafter
(c) determining the presence or absence of a first color or a first fluorescence in said mixture;
(d) adding an indole detection reagent to said mixture; thereafter
(e) determining the presence or absence of a second color in said mixture;
wherein the presence of said first color or said first fluorescence in said mixture indicates the presence of a coliform bacterium in said sample and the presence of said second color in said mixture indicates the presence of *E.coli* in said sample.

In a more preferred embodiment, the invention provides a method for specifically detecting coliform bacteria and *E.coli* in a water sample, which method comprises steps:
(a) forming an aqueous sample-medium mixture by contacting said sample with a medium comprising:
   (i) tryptophan;
   (ii) ortho-nitrophenyl-β-D-galactopyranoside;
   (iii) a protein or peptide hydrolysate in an amount sufficient to promote the growth of *E.coli;*
(b) incubating said mixture at a temperature of 44.5°C for 2 to 24 hours; thereafter
(c) determining the presence or absence of a yellow color in said mixture;
(d) adding p-dimethylaminobenzaldehyde in acidified n-butyl alcohol or acidified isoamylalcohol to said mixture; thereafter
(e) determining the presence or absence of a red-purple color in said mixture;
wherein the presence of said yellow color in said mixture indicates the presence of a coliform bacterium in said sample and the presence of said red-purple color in said mixture indicates the presence of *E.coli* in said sample.

### BRIEF DESCRIPTION OF DRAWINGS

Figure 1 is a view of E.coli (ECOTAG) test tubes after 18 hours of incubation.

Figure 2 is a view of coliform (COLITAG) test tubes after 18 hours of incubation.

### DETAILED DESCRIPTION OF THE INVENTION

The current invention is based on finding that E. coli is the only major group of Enterobacteriaceae bacteria which possess two most definitive traits of their species, namely lactose utilization and indole production. By combining the method for lactose utilization measurement with the method for measurement of indole production, a novel, improved, fast, sensitive, specific, inexpensive, reliable and accurate test has been developed for determination of E. coli and coliform bacteria in water.

The current invention is based on the observation that tryptophanase, the enzyme responsible for indole production, is strongly repressed in the presence of lactose (Abstract: Ann. Meet. Amer. Soc. Microbiol., Q12 (1990)). Therefore, one can avoid this--repression by simply omitting lactose from the medium and replacing it with a suitable synthetic β-D-galactopyranoside such as ONPG (in this application, the term "galactoside" and "galactopyranoside" is used interchangeably). ONPG serves to indicate the ability of a bacterium to utilize lactose. However, unlike lactose, it does not repress tryptophanase or indole production. Therefore, indole production can be assessed by simply adding a suitable reagent such as Kovacs' or Ehrlich's reagent and looking for the characteristic color change.

The current invention is also based on recognition of the role of catabolite repression. Catabolite repression by a readily available carbohydrate energy source, such as lactose or lactose substitute in large excess, impairs the metabolism, conversion and utilization of a less efficient energy source, in this case tryptophan.

The current invention utilizes a medium which avoids such catabolic repression by introducing multiple indicator media TAG (Tryptophan-Galactoside), which utilize substitute energy sources in minimal amounts which will not cause or allow such catabolite repression.

The current invention enables one to detect the presence of coliform bacteria and E. coli simultaneously in water samples. It can be used as a rapid one-tube test, it does not require isolation of a pure culture or production of fully-grown colonies and it is able to determine the presence of even one singe E. coli bacteria.

This invention, consequently, is based on the observation that the amino acid tryptophan is metabolized into indole, pyruvic acid and ammonia and that indole is produced in equimolar amount to that of ammonia. The test is fast, it takes 2-24 hours, depending on the degree of contamination to conclusively determine the presence or absence of E. coli; it is accurate, as it determines with almost 100% accuracy whether there are or there are not E. coli present in the water sample without giving false negative or positive readings; it is sensitive, as it can determine, by using a dilution method, a most probable number of contaminating E. coli and even one single E. coli bacteria in water; and it is specific to E. coli, since it is able to distinguish between unharmful coliforms and E. coli. The test simultaneously determines the presence of β-galactosidase, an enzyme evidencing lactose utilization, and tryptophanase, an enzyme evidencing formation of indole. Using this test, only those bacteria which simultaneously produce indole and utilize lactose give positive results. In water samples, such ability is present essentially only in the bacteria E. coli.

The test is specific to those Enterobacteriaceae which can simultaneously produce indole and ferment lactose and thus are able to grow and generate color and/or fluorescence in a specific medium containing minimal amounts of tryptophan and galactoside. In water samples, these Enterobacteriaceae are overwhelmingly E. coli.

Indole production is generally suppressed by glucose, lactose or by any other readily utilized sugar. Consequently, the indole test does not work in the presence of lactose known to be a substrate involved a normal metabolic pathway of E. coli.

It is a well known fact that when E. coli are placed in a culture medium containing lactose, an inducible enzyme β-galactosidase is synthesized in a large amount. The induced β-galactosidase hydrolyzes lactose (I) according to Reaction Scheme I, to products D-glucose (II) and D-galactose (III) that can be used directly as fuel and carbon source.

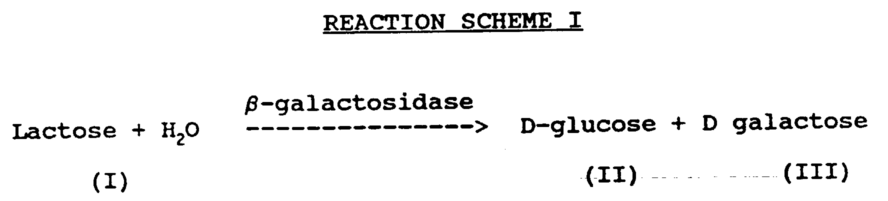

This distinct characteristics of E. coli is used as one of the primary features of this invention which enables quick and accurate separation of Enterobacteriaceae having this characteristic from those which do not possess it. In the test, the ability to utilize lactose is detected by the hydrolysis of chromogenic or fluorogenic β-D-galactosides.

The second distinct characteristic of E. coli is its ability to utilize tryptophan as a nitrogen source and to metabolize it with the enzyme tryptophanase. The reaction is detected with the indole test. The basis of the indole test is the reaction illustrated in Reaction Scheme II. Tryptophan (IV) is metabolized with E. coli tryptophanase into free indole (V), pyruvate (VI) and ammonium (VII).

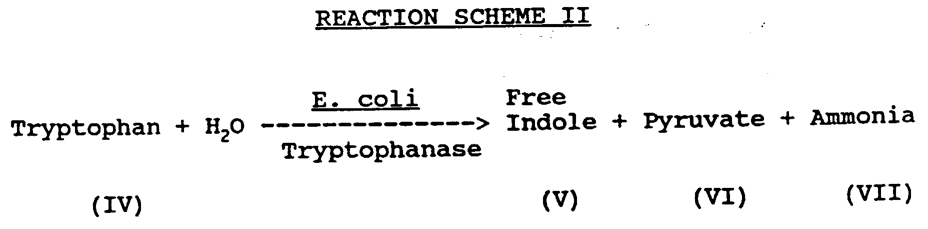

Both the first and second distinct characteristics of E. coli are based on metabolic flexibility of E. coli cells. E. coli are chemoorganotrophics, in that they require an organic source of carbon and metabolic energy, however, within this category they are metabolically versatile. As shown in case of lactose, when there is no glucose present and readily available as an energy and carbon source, E. coli utilizes lactose and quickly reorients its enzymatic synthesis to produce enough β-galactosidase to suffice to metabolize lactose to D-glucose and D-galactose.

Similarly to the lactose as the carbon source, E. coli can also utilize various sources of nitrogen other than ammonia. In fact, when E. coli cells are provided with an ample supply of exogenous amino acid, they stop synthesizing amino acids de novo from ammonia and use the preformed acids instead.

In lieu of lactose or glucose, E. coli can also use certain amino acids as fuel and carbon sources when necessary. The current invention recognizes this propensity of E. coli and advantageously utilizes the amino acid tryptophan which contains in its molecule an indole. Tryptophan can be metabolized by the specific enzyme tryptophanase to free indole, pyruvate and ammonium. As there are very specific tests available for determination of indole, the current invention utilizes this feature to isolate E. coli, from all other bacteria which do not possess both lactose fermentation and indole formation.

Since, however, it is well known that indole formation is inhibited in the presence of lactose fermentation, this invention utilizes the lactose surrogate to eliminate such metabolic inhibition.

Based on the above background, in practice, the current invention is based on determination of the presence of the enzyme β-galactosidase and on the presence of free indole.

As describe above, when an ample amount of lactose is present in the medium, lactose is catalyzed by and induces the production of the enzyme β-galactosidase, which has distinct chemical properties and can be easily measured or otherwise determined. However, since lactose is known to inhibit production of indole from tryptophan, then clearly, indole cannot be produced and measured when lactose is present. By substituting lactose-with a surrogate compound which is also metabolized by β-galactosidase, the inhibitory effect of lactose is removed and indole will form and may be measured simultaneously with the lactose-surrogate's utilization.

This invention further advantageously utilizes the fact that E. coli is a convertible organism which can respond quickly to all of its metabolic demands. Thus, if the specific lactose-surrogate which can be measured is offered as a sole carbon source, E. coli will quickly respond by immediate production of β-galactosidase. However, if all other more easily utilized carbon sources are eliminated from the media, the E. coli metabolic regulation system will recognize tryptophan as a carbon source. Thus, if the tryptophan is offered and all other carbon sources are either completely eliminated or controlled in such a way that they are advantageously utilized in the test, then tryptophanase is formed by E. coli in a large amount for conversion of tryptophan into easily measurable free indole. In testing the current invention, three basic bacteriological experimental setups were tested with internal variations.
- Setup 1:: Control samples containing Salmonella but no coliform or E. coli bacteria.
- Setup 2:: Experimental samples containing only E. coli bacteria. This group contained various types of E. coli, such as E .coli ATCC 25922, ECOR 13, ECOR 8, ECOR 5, ECOR 4, ECOR 6, ECOR 47, ECOR 13, all obtained from American Type Culture Collection. TC 244, TC 234; TC 218, TC 217, internally isolated at University of California, Berkeley or PF 226, PF 222 obtained from Peter Feng, FDA Laboratory.
- Setup 3:: Experimental samples containing other coliform bacteria such as TC 257, Klebsiella Pneumoniae, Enterobacter cloacae, or Citrobacter freundii.

Salmonella typhimurium, obtained from B. Ames, is normally both lactose and indole-negative. E. coli TC 224, 234, 218, 217 are indole and lactose positive. E. coli PF 222 and 226 are indole positive and lactose positive.

For a quick test for the presence and absence of E. coli, setups 1, 2 and 3 were used. For total coliform and E. coli, setups 1, 2 and 3 were used. For semiquantification, either setup may be used.

Various media bases were prepared and tested. The basic requirement for the media base was that it contains at least an optimal minimum amount of tryptophan or tryptophansubstitute and an optimal minimum amount of lactose-surrogate.

One of the most important features of the current invention is the new type of medium containing tryptophan and β-galactoside specifically designed to avoid the catabolite repression by its own ingredients, Such catabolite repression happens when there is an excess of readily available carbohydrate energy source (lactose surrogate) which- represses metabolism, conversion and utilization of a less efficient energy source (tryptophan).

The uniqueness of the current media stems from its composition of ingredients balanced to avoid and eliminate such catabolite repression. Thus, when MMO-MUG or other currently available media are-used, they catabolically repress the second unique feature of E. coli, namely indole formation, by the β-glucuronidase or other lactose substitute supplied in excess.

The current TAG medium, on the other hand, provides balanced energy sources so that both the lactose utilization and indole formation can proceed at the same time, and thus be determined at the same time in the same tube. Consequently, TAG medium contains essentially the lactose substitute and the indole source. Also added are the growth promoting components and other components.

The second most important requirement for the medium used in the practice of the current invention is that it is not growth limiting but rather that it is growth promoting. To that end, the current TAG medium contains the growth supporting and promoting component protein or peptide hydrolysate such as tryptose and may also contain yeast extract.

These components are added in quantities sufficient to promote and support growth of E. coli, so that another feature of the current invention is a significant promotion of the growth of E. coli, allowing the detection of even a small number of E. coli, and in many cases even of one single E. coli cell present in a highly diluted water sample. This can be achieved because E. coli growth is promoted by substitute alternate nutrient components such as yeast extract or tryptose. Thus, the growth of the E. coli in the current TAG medium is not limited by the availability of the ONPG and MUG as the nutrient as in the other currently available media.

By deliberately promoting growth of E. coli with tryptose, the current invention utilizes a medium, or family of media, that is very efficient at recovering a small number or single E. coli cells.

Comparative results obtained by using the standard EC medium, the MMO-MUG (Colilert) medium and the TAG (Colitag) medium are shown in Tables 1 and 2 and discussed in Example 9.

The ability to test samples with minimal number of E. coli present and to obtain reliable results, i.e., a positive reaction from a single bacterial E. coli cell is very important feature in water testing where the contaminant's dilution is so extreme that a culture or bottle may contain only one or few E. coli cells.

Still another important improvement against other known E. coli detection methods is the time saving achieved with the method of this invention.

The medium of the current invention gives faster responses than any other, including MMO-MUG medium. That is particularly true when one tests highly diluted water samples which contain only one or few E. coli cells. In such intances, because of the growth promoting components present in the media, one can obtain the positive or negative answer in 2-24 hours, as compared to 24-48 hours or longer needed for other tests.

The current media deliberately uses a balance of components which promote and support growth of the E. coli cells but at the same time avoid and suppress the catabolite repression. These features make it-possible to combine carbohydrate utilization using lactose substitutes (β-galactosides) and indole formation using secondary nitrogen sources, allowing testing of both features characterizing the presence of E. coli, namely lactose utilization and indole production. Since under normal circumstances lactose fermentation suppresses indole production, because the tryptophan is a lower priority energy provider, in other tests the lactose fermentation effectively eliminates and suppresses the metabolism of tryptophan to indole, and the presence of indole cannot be measured-at- the same time in the same tube.

As the lactose-substitutes, the nonfluorescent compounds ONPG, indolyl-β-D-galactoside, X-galactoside (5-bromo, 6-chloro-indolyl-β-D-galactoside (X-GAL)), 8-hydroxyquinoline-β-D-galactopyranoside (HQDG) and other known lactose substitutes in an amount from 0.05-10 g/l, preferably 0.5 g per liter of normal solution and from 0.1-30/1, preferably 1.5 g per liter of concentrated solution, or fluorescent methylumbellyferyl-galactoside (MU-GAL) substitute in an amount from 0.05-5g, preferably 0.25 g/l were used, preferably ONPG or MU-GAL.

Nonfluorescent lactose substitutes normally produce a yellow color (ONPG) or blue color (X-GAL or indolyl-β-D-galactoside). Hydroxyquinoline produces intense black pigment in the presence of any ferric salt such as ferric chloride. MU-GAL produces fluorescence visible under UV.

As the indole source confering a red color on E. coli, tryptophan, and other indole sources or non-indole tryptophan analogues such as S-o-nitrophenyl-1-cysteine (SOPC) which gives, when ionized, a bright yellor color but when deprotonated is colorless, are used. Tryptophan is used, as the preferred source, in amount from 0.1 to 20 g/l, preferably 1 g per liter of normal solution and 0.1 to 30 g/l, preferably 3 g per liter of concentrated solution. O-nitro-thiophenol derived from tryptophan will produce red color. Indole derived from SOPC produces yellow color but when used in combination with X-galactoside, which produces blue color, the resulting color is green. Thus, it is possible to design and choose a color- of the test by substituting tryptophan with SOPC.

The medium contains as a growth promoting component hydrolyzed protein or peptide mixture, preferably tryptose or any other nitrogen containing broth as an additional source of nitrogen, in amount from 0.05-30 g/l, preferably from 3-30 g per liter of concentrated solution. Additionally, yeast may be added in amount from 10 mg-10 g/l, preferably 50 mg/l.

Additionally, β-galactosidase enzyme inducer such as isopropyl-β-D-thiogalactopyranoside (IPTG) , methylthiogalactoside, melibiose in amount from 0.01 to 1 g/l, preferably 0.1 g/l of normal medium base and from 0.03 to 3 g/l, preferably 0.3 g per liter of concentrated medium may be added. To inhibit the growth of interfering bacteria, the medium may optionally also include bacteria growth inhibitors such as sodium lauryl sulfate, bile salt, detergents, tergitol-7, antibiotics Monensin, Nalidixic acid or some such others, in an amount from 0.01 to 1 g/l, preferably 0.1 g per liter of normal medium and from 0.03 to 3 g/l, preferably 0.3 g per liter of concentrated medium.

To prepare the TAB medium, the above ingredients are dissolved in 1 liter of sterile fluid, such as distilled or deionized water. To the final medium, additional amounts of sodium chloride or any other suitable salt such as sodium diphosphate, magnesium sulfate, potassium diphosphate, sodium lauryl sulfate, magnesium sulfate, disodium phosphate, monosodium glutamate and others, in amount from 0.01 to 10 g/liter, non-toxic to bacteria may be added up to 50 mmol concentration.

Various bases were prepared and tested.

### 1) Normal TAG Medium Base

1 g of tryptophan and mineral salts, disodium phosphate (3 g), magnesium sulfate (0.1 g), potassium diphosphate (0.5 g) were dissolved in distilled water. The solution was sterilized at 121°C for 15 minutes. ONPG was dissolved in distilled water at 5 g/l, filter sterilized at room temperature, and diluted 10-fold with the tryptophan salts solution. The resulting stock solution was stored in the refrigerator at 2-8°C until used.

### 2) Improved TAG Medium Base

The Normal TAG Medium Base was supplemented with 0.1 g/l of IPTG which was added as a filter-sterilized solution. Solution was gently mixed to achieve dissolution of all components. Until used, the medium was stored in the refrigerator.

### 3) 3x Concentrated TAG Medium Base

This medium contains 3 g of tryptophan, 1.5 g of ONPG, 0.3 g of IPTG, and 3-fold higher levels of mineral salts than the Normal TAG Medium Base. Tryptophan and salts were heat-sterilized. ONPG and IPTG were filter-sterilized. The medium was subsequently stored in the refrigerator.

### 4) Sodium Lauryl Sulfate TAG Medium Base

1 g of tryptophan, 0.5 g of ONPG, 0.1 g of sodium lauryl sulfate, 0.1 g of isopropylthio-β-D-galactopyranoside and sodium chloride up to 50 mmol, and disodium phosphate, potassium diphosphate and magnesium sulfate were prepared as described above. The sodium lauryl sulfate was mixed with tryptophan and other heat-stable ingredients before heat-sterilization.

### 5) Concentrated TAG Medium Base

1 g of tryptophan, 0.5 g of ONPG, 3 g of tryptose, 0.1 g of sodium lauryl sulfate, 0.1 g of isopropyl-β-D-thiogalactopyranoside and sodium chloride up to 50 mmol, are dissolved in 1000 ml of 0.85% sodium chloride solution (saline).

### Other Variations of TAG Medium

6) 1 g of tryptophan, 0.1 g of magnesium sulfate, 0.5 g of potassium diphosphate, 3 g of disodium phosphate and 0.1 g sodium lauryl sulfate is dissolved in 1000 ml of distilled water. The solution is autoclaved at 121°C for 15 minutes and 0.5 g of filter sterilized ONPG is added.
7) 1 g of tryptophan, 0.1 g of magnesium sulfate, 0.5 g of potassium diphosphate, 3 g of disodium phosphate, 0.1 g sodium lauryl sulfate and 50 mg of yeast extract is dissolved in 1000 ml of distilled water. The solution is autoclaved at 121°C for 15 minutes and 0.5 g of filter sterilized ONPG is added.
8) 1 g of tryptophan, 0.1 g of magnesium sulfate, 0.5 g of potassium diphosphate, 3 g of disodium phosphate, 0.1 g sodium lauryl sulfate, and 20 g of tryptose is dissolved in 1000 ml of distilled water. The solution is autoclaved at 121°C for 15 minutes and 0.5 g of filter sterilized ONPG is added.
9) 1 g of tryptophan, 0.1 gof magnesium sulfate, 0.5 g of potassium diphosphate, 3 g of disodium phosphate, 0.1 g sodium lauryl sulfate, and 0.5 g of monosodium glutamate is dissolved in 1000 ml of distilled water. The solution is autoclaved at 121°C for 15 minutes and 0.5 g of filter sterilized ONPG is added.
10) 1 g of tryptophan, 0.1 g of magnesium sulfate, 0.5 g of potassium diphosphate; 3 g of disodium phosphate, 0.1 g sodium lauryl sulfate, 0.25 ammonium sulfate, and 0.5 g of monosodium glutamate is dissolved in 1000 ml of distilled water. The solution is autoclaved at 121°C for 15 minutes and 0.5 g of sterilized ONPG is added.
11) 1 g of tryptophan, 0.1 g of magnesium sulfate, 0.5 g of potassium diphosphate, 3 g of disodium phosphate, 0.1 g sodium lauryl sulfate, and 0.25 ammonium sulfate is dissolved in 1000 ml of distilled water. The solution is autoclaved at 121°C for 15 minutes and 0.5 g of sterilized ONPG is added.
12) 1 g of tryptophan, 0.1 g of magnesium sulfate, 0.5 g of potassium diphosphate, 3 g of disodium phosphate, 0.1 g sodium lauryl sulfate, and 100 mg of-yeast extract is dissolved in 1000 ml of distilled water. The solution is autoclaved at 121°C for 15 minutes and 0.5 g of sterilized ONPG is added.
13) 1 g of tryptophan, 0.1 g of magnesium sulfate, 0.5 g of potassium diphosphate, 3 g of disodium phosphate, 0.1 g sodium lauryl sulfate, and 25 mg of yeast extract is dissolved in 1000 ml of distilled water. The solution is autoclaved at 121°C for 15 minutes and 0.5 g of sterilized ONPG is added.
14) 1 g of tryptophan, 0.1 g of magnesium sulfate, 0.5 g of potassium diphosphate, 3 g of disodium phosphate, 0.1 g sodium lauryl sulfate, and 5 g of tryptose is dissolved in 1000 ml of distilled water. The solution is autoclaved at 121°C for 15 minutes and 0.5 g of filter sterilized ONPG is added.
15) 1 g of tryptophan, 0.1 g of magnesium sulfate, 0.5 g of potassium diphosphate, 3 g of disodium phosphate, 0.1 g sodium lauryl sulfate and 2.5 g of tryptose is dissolved in 1000 ml of distilled water. The solution is autoclaved at 121°C for 15 minutes and 0.5 g of filter sterilized ONPG is added.

### 16) TAG Stock Solution

0.285 g of tryptophan, 0.855 g of disodium phosphate, 0.1425 g of potassium diphosphate, 0.0285 g of sodium lauryl sulfate, 0.0285 of magnesium sulfate, and 0.7125 g of ammonium sulfate is dissolved in 285 ml of saline and used as a stock solution.

### 17) TAG-YEAST Stock Solution

To 50 ml of TAG stock solution is added 0.05 g of yeast extract to obtain yeast stock of 1 g/l. This was diluted 10-fold for use.

### 18) TAG-ONPG solution

To 25 ml of TAG stock solution (16), 0.125 g of ONPG is added. The solution is filter sterilized.

The stock solutions were prepared as a convenient base TAG solutions which were used for preparation of many specific TAG solutions tested during the optimization of TAG medium.

These and other variations of the TAG media based on the above-listed ingredients or other ingredients which would satisfactorily substitute one or more of ingredients above listed, are contemplated to be within the scope of this invention.

TAG medium base solutions are sterilized either by autoclaving, by micropore filtration, irradiation or by any other method suitable for such purposes, which method does not damage individual ingredients. The sterilization can be achieved by using sterile glassware, solution and ingredients, or a partial mix may be autoclaved, or complete TAG medium may be sterilized by filtration.

The stock solutions or ready made solutions are preferably stored at 2-8°C in the refrigerator or they may be freeze-dried, dry-blended and irradiated, vacuum dried, lyophilized or preserved in any other suitable manner recognized in the industry.

The ingredients of any TAG medium or similar medium nay also be mixed as a dry mixture or in any suitable volatile organic or inorganic solvent. Sterilization may be achieved by irradiation or by the solvent itself.

When used for bacteriological testing for presence of E. coli, an appropriate amount of the sterile TAG medium is mixed with a water sample to be tested in amount from 1:10 to 10:1 ratio, preferably 2:1 ratio, of water sample to TAG medium. The solution is gently swirled or mixed and preferably incubated at 35°C for 4 hours and at 44.5°C for an additional time up to 24 hours. When the incubation of the mixture is done at higher temperatures than 50°C, weak or injured E. coli may not survive and the results may be false negative. When the incubation is done solely at lower temperatures such as 35°C, false positive results for indole are obtained. The samples are incubated for 2 to 24 hours. If there is no color developed at 24 hours, there is no E. coli present. Normally, light E. coli contamination may be noticeable as early as in 2 hours, usually at 15-18 hours, latest at 24 hours. On the other hand, a water sample contaminated with large numbers of coliforms or E. coli will color the medium fairly soon and positive results may be obtained as soon as around 2-6 hours, when incubated wt 44.5°C.

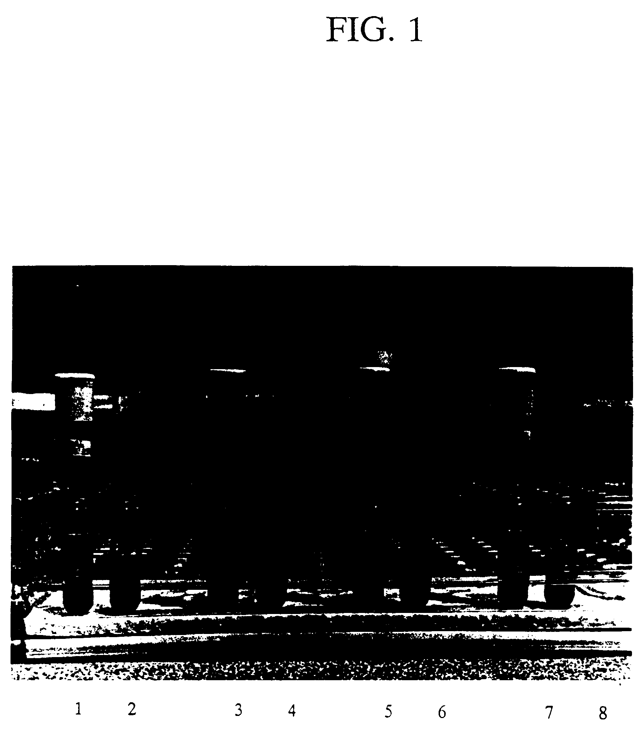

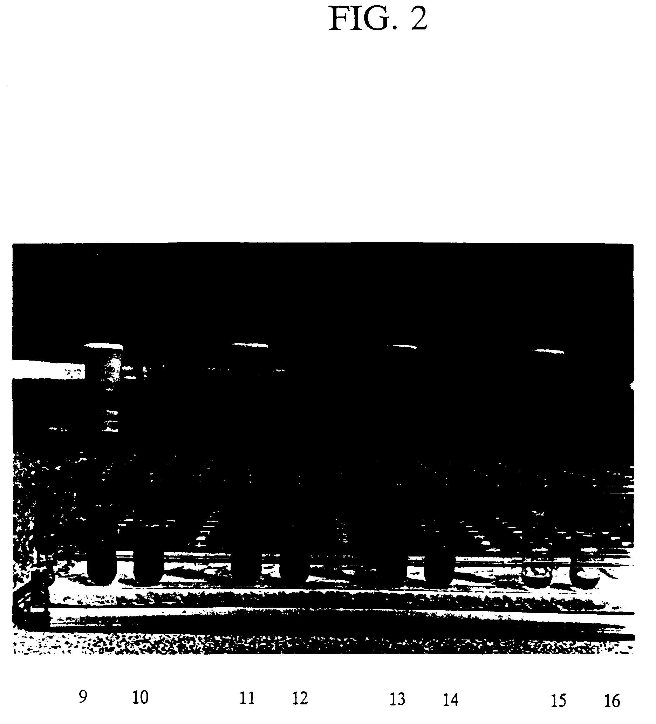

The tests for determination of total coliform and of E. coli were named COLITAG™ and ECOTAG™. Both tests are illustrated in Figures 1 and 2. Figure 1 (Setup 2, page 21) shows the results of the ECOTAG test run on four strains of E. coli, namely E. coli strain ATCC 25922-tubes 1 and 2, ECOR 4-tubes 3 and 4, ECOR 5 - tubes 5 and 6, ECOR 6-tubes 7 and 8. All tubes were incubated for 18 hours at 35°C after mixing the water sample containing E. coli with the TAG Medium Number 3. The yellow color developed in aqueous lower phase in tubes 1, 3, 5 and 7 indicates β-galactosidase activity. After addition of 2 drops of Kovacs' reagent, reddish-purple ring on the top of the solution (organic phase in tubes 2, 4, 6 and 8) shows the presence of indole. Indole production indicates the presence of indole-positive coliforms. In water, most indole positive coliforms are E. coli.

In comparison, in Figure 2 (Setup 1 and Setup 3 pages 14 and 15), the samples in tubes 9-14 are non-E. coli coliforms. Specifically, in tubes 9 and 6 are Enterobacter cloacae, in tubes 11 and 12 are Citrobacter freundii, and in tubes 3 and 14 are Klebsiella pneumoniae. In tubes 15 and 16 are noncoliform Salmonella. All these bacteria are indole negative and when submitted to the same testing procedure as described for Figure 1, the yellow color was seen in tubes 9, 11 and 13 similar to the yellow color in tubes 1, 3, 5 and 7. When the Kovacs reagent was added to tubes 10, 12 and 14, there was no color change. The yellow color in the organic phase indicated the absence of the indole formation, showing that there were no indole-positive bacteria present and that all bacteria present in tubes 9-14 were indole-negative, i.e., coliform but not E. coli. As a control for testing the accuracy of both the COLITAG and ECOTAG tests, non-coliform bacteria Salmonella was tested using the same procedure. The liquid in tubes 15 and 16, as can be seen is colorless, and does not show the yellow color for coliform, nor the red-purple color for E. coli.

The colorimetric results given by both tests COLITAG and ECOTAG, using the TAG medium containing tryptophan and ONPG can be summarized as follows.
Colorless result in both aqueous and organic phases indicates the absence of both E. coli and coliform.
Yellow color in the aqeous phase indicates the presence of coliform which may or may not include E. coli.
Yellow color in the organic phase following the reaction with the Kovacs reagent indicates the absence of E. coli.
Red-purple color in the organic phase following the reaction with the Kovacs reagent indicates the presence of E. coli.

In the medium, if X-GAL is exchanged for ONPG, then instead of the yellow color, a blue color is obtained indicating the presence of coliform.

In the medium variation containing MU-GAL instead of ONPG, the samples containing coliform are fluorescent under UV light.

In the medium variation containing HQDG and ferric chloride, the presence of coliform is indicated by black pigment.

In the medium where X-GAL is used instead of ONPG and SOPC is used instead of tryptophan, a green color evidences the presence of E. coli. SOPC produces yellow color, X-GAL produces blue color. Combined, they give a green color indicating the presence of E. coli.

The ECOTAG test may further be used for enumeration of the number of E. coli present. Typically, serial dilution in saline or in 0.1% peptone of the water sample would be made, as described in Example 5, and then the ECOTAG Test would be run. The presence of the yellow color would be recorded in all dilutions. Results would be calculated using the Most Probable Numbers table, according to methods known and accepted in the art.

### MATERIALS

Ehrlich's Reagent, p-dimethylaminobenzaldehyde in acidified n-butyl alcohol (Sigma) ; Kovacs Reagent, p-dimethylaminobenzaldehyde in acidified isoamylalcohol (Sigma); tryptophan (Sigma); tryptose (Difco), o-nitrophenyl-β-D-galactopyranoside (Sigma) sodium lauryl sulfate (Sigma); isopropyl β-D-thiogalactopyranoside (Sigma); yeast extract (Difco), L-glutamate (or Sigma Cal Biochem).

### EXAMPLE 1

### A Method for Determination of Presence or Absence of Total Coliform and E. coli in Water Samples

This example illustrates the method for quick visual "COLITAG" test determination of presence or absence of E. coli and/or coliform in water samples. The method utilizes distinct characteristics specific to coliform and E. coli., namely the ability to utilize lactose only (coliform) and both a lactose utilization and formation of indole (E. coli).

The principle of this method is that the lactose which normally inhibits formation of indole, is substituted with another substrate which is metabolized or utilized in the same manner as lactose, by the enzyme β-galactosidase. The substitute for lactose does not inhibit formation of indole from tryptophan. Consequently, when the yellow color develops, the sample contains coliforms and may or may not contain E. coli. When there is no yellow color present, there is no coliform and no E. coli present. When the yellow color develops and also the purple color is present after addition of Kovac reagent, E. coli is present.

For this method, a specific tryptophan-galactosidase (TAG) medium containing minimal amounts of tryptophan and lactose-substitute ONPG was prepared and used. Medium optionally has isopropyl-β-D-thiogalactopyranoside (IPTG), lactose production inducer, and is prepared in distilled water.

### Preparation of Normal TAG Medium (1)

1 g of tryptophan and mineral salts, disodium phosphate, magnesium sulfate, potassium diphosphate were dissolved in distilled water. The solution was sterilized at 121°C for 15 minutes. ONPG was dissolved in distilled water at 5 g/l, filter sterilized at room temperature, and diluted 10-fold with the tryptophan salts solution. The resulting stock solution was stored in the refrigerator at 2-8°C until used.

### Preparation of Improved TAG Medium Base (2)

1 g of tryptophan, potassium diphosphate, 3g disodium phosphate, 0.1 G sodium lauryl sulfate, 2.9 g sodium chloride, and 1.0 g tryptose were dissolved in 1000 ml of distilled water and sterilzed at 121°C for 15 minutes. Then 0.5 g ONPG and 0.1 g IPTG were added as a filter-sterilized solution. Until used, the medium was stored in the refrigerator.

### Preparation of Concentrated TAG Medium Base (3)

All the ingredients of the above medium were prepared at 3 times the usual concentration. For example, 3 g of tryptophan, 1.5 g of ONPG and 0.3 g of IPTG. The components were mixed, sterilized by filtration, and stored as above.

### Procedure for determination of E. coli

Figures 1 and 2 illustrate this example. Into pre-sterilized 20 ml bottles containing 3 ml of concentrated TAG medium was inoculated with 1000 bacterial cells in 0.010 ml 0.85% saline sample to be tested.

Samples 1-8 were E. coli:
1 and 2 were ATCC 25922
3 and 4 were ECOR 4
5 and 6 were ECOR 5
7 and 8 were ECOR 6

Samples 9 -14 were Non-E. coli coliform:
9 and 10 were Enterobacter cloacae
11 and 12 were Citrobacter freundii
13 and 14 were Klebsiella pneumoniae

Samples 15 and 16 were non-coliform bacteria:
15 and 16 were Salmonella

The bottles were closed and gently shaken so that the samples and the medium were properly mixed. The bottles were put in the water bath at 35°C for 4 hours incubation, followed by 20 hours incubation at 44.5°C. Following the incubation, samples were visually examined.

### Results:

Control Samples 15 and 10 remained colorless, there were no coliforms and E.coli present. When the aqueous phase of samples 1-8 was bright yellow showing the presence of β-galactosidase, E. coli seemed to be present and had grown to large numbers. The aqueous phase of coliform samples 9-14 was also bright yellow indicating the presence and subsequent growth of coliform bacteria.

Indole testing done on every other tube (i.e., tubes 2, 4, 6 and 8) with a few drops of Kovacs reagent indicates that samples 2, 4, 6 and 8 contained E. coli. The purple color indicates the presence of indole (Figure 1).

The aqueous phase in samples 9-14 is yellow (Figure 2), thus indicating a presence of β-galactosidase activity. When the Kovacs reagent was added to tubes 10, 12, 14 and 16, there was no purple color. This shows that these bacteria were not E. coli because there was no formation of indole.

Tubes 15 and 16 were non-coliform bacteria which did not show the yellow or purple color and thus did not contain neither coliform, nor E. coli.

This is a coliform and E. coli presence/absence method. The yellow color and indole test indicates that both lactose utilization and indole production characteristic for E. coli were present in samples 1-8. The yellow color and negative indole tests of samples 9-14 evidenced the presence of coliforms other than E.coli.

### EXAMPLE 2

### "ECOTAG" QUICK TEST - A KIT FOR DETERMINATION OF COLIFORM AND E. COLI IN WATER SAMPLES

### SUMMARY

E. coli presence in the water is generally recognized as evidence of sewage contamination.

E. coli is a species of family Enterobacteriaceae. E. coli possess two characteristics: lactose utilization, and indole production distinguishing it from other Enterobacteriaceae and other coliforms.

In detection of E. coli, ECOTAG QUICK TEST utilizes these two characteristics and determines the presence of β-galactoside, an enzyme involved in lactose utilization and the presence of tryptophanase, an enzyme involved in indole production. In detection of E. coli presence, water samples are reacted with ECOTAG QUICK TEST medium containing tryptophan and o-nitrophenyl-β-galactopyranoside (ONPG) at 35°C for 4 hours, followed by 44.5°C for 20 hours or until a yellow color develops. The presence of the yellow color indicates a lactose utilization. Indole formation is determined with Kovacs reagent as a red-purple color. The presence of the yellow color in water phase indicates lactose utilization and the presence of red-purple color indicate the indole formation and both indicates the presence of E. coli in tested water sample.

### PRODUCT DESCRIPTION, STORAGE

ECOTAG QUICK TEST consisting of:

3 sterile glass vials having a line at 100 ml level with stoppers of 200 ml volume containing 50 ml of concentrated freeze-dried TAG Medium each.

Concentrated TAG Base Medium contains 3 g of tryptophan, 1.5 g of ONPG, 0.3 g of isopropyl-β-D-thiogalactopyranoside (ITPG) 9 g sodium chloride, 0.3 g magnesium sulfate, 1.5 g potassium diphosphate, 9 g disodium phosphate, and 0.9 g sodium lauryl sulfate dissolved in distilled water. Sterilized.

Vial with Kovacs reagent with droppers.

Store the ECOTAG QUICK TEST in the refrigerator at 2-8°C until used. Do not open glass vials until ready to use.

### SPECIMEN

Water sample to be tested-undiluted.

### TEST PROCEDURE

Principle: Tryptophan in the medium provides a nitrogen source for E. Coli and is metabolized by E. coli tryptophanase to form free indole, pyruvate and ammonia in equimolar amounts. ONPG substituting for lactose is metabolized with β-galactosidase. At 35°C E. coli grow and utilize ONPG and produce indole, at 44.5°C, all other indole positive non E. coli bacteria are eliminated. β-galactoside turns the medium yellow. Since the tryptophan is used as the sole nitrogen source, no other bacteria than coliform and E. coli will produce the yellow color under these conditions. Upon addition of Kovac's reagent, the solution turns purple-red when E. coli is present.

### SET-UP

Remove the vials with medium from the refrigerator, open the vial and add the water sample up to 100 ml mark on the vial. Close the vial with the stopper, swirl the vial gently so that the medium and water mix. Incubate at 35°C for 4 hours and at 44.5°C for another 20 hours until a bright yellow color develops or 24 hours whatever is shorter. Record results. Add 2-3 drops of Kovacs reagent, mix, let stand for 1 hour. Observe. Record purple-red color which may develop immediately.

### EVALUATION OF RESULTS

Bright yellow color of the solution signifies the presence of coliform. The red-purple color signifies the presence of E. coli. No change in color signifies the absence of E. coli and coliform.

### EXAMPLE 3

### Method for Determination of Total Coliform and E. Coli "COLITAG"

This example illustrates the determination of total coliform and E. coli present in diluted water samples by utilizing tryptophan as one nitrogen source, another nitrogen containing compound as the second nitrogen source and E. coli growth promoter and lactose substitute for determination of β-galactosidase. Under these conditions, most Enterobacteriaceae grow, all coliforms give yellow color and E. coli, distinguished by indole production, give red-purple color with either Kovac's or Ehrlich reagent.

### Preparation for Normal TAG Medium Base (4)

1 g of tryptophan, 0.5 g of ONPG, 1 g of tryptose, 0.1 g of sodium lauryl sulfate, 0.1 g of isopropyl-β-D-thiogalactopyranoside and sodium chloride up to 50 mmol, 0.1 g MgSO₄, 0.5 g KH₂PO₄, 3 g Na₂HPO₄ are dissolved in 1000 ml of distilled water and sterilized at 121°C for 15 minutes. The ONPG and IPTG are added as a filter sterilized solution.

### Preparation of Concentrated TAG Medium Base (5)

This medium is like the Normal Medium except that all ingredients are 3 times as concentrated. For use in Presence-absence tests, this medium is prepared in 50 ml portions in sterile bottles. Then 100 ml of water sample are added, the sterile caps reapplied, and the bottles incubated at 35°C for 4 hours, followed by 44.5°C for 20 hours.

### Procedure for determination of total coliform and E. coli

The procedure is illustrated in Figures 1 and 2.

Sixteen sterile glass 30 ml vials were prepared and marked 1-16. Into these pre-sterilized vials containing 30 ml of freeze-dried concentrated TAG medium base (5) was inoculated with 1000 bacterial cells in 0.010 ml 0.85% saline sample to be tested.

Samples 1-8 were E. coli:
1 and 2 were ATCC 25922
3 and 4 were ECOR 4
5 and 6 were ECOR 5
7 and 8 were ECOR 6

Samples 9 -14 were Non-E. coli coliform:
9 and 10 were Enterobacter cloacae
11 and 12 were Citrobacter freundii
13 and 14 were Klebsiella pneumoniae

Samples 15 and 16 were non-coliform bacteria:
15 and 16 were Salmonella

The bottles were closed and gently shaken so that the samples with the medium were properly mixed. The bottles were put in the water bath at 35°C for 4 hours, followed by 20 hours 44.5°C incubation. Following the incubation, next morning the samples were visually examined.

### Results:

Control samples 15-16 remained colorless showing that neither coliform nor E. coli were present. Coliform only samples 9-14 were bright yellow showing that various coliform were present, however, at this point it was not clear whether any E. coli were present in samples 9-14. Samples 1-8 containing E. coli only were also bright yellow suggesting that there were either coliform and/or E. coli. Again, at this point it was unclear whether only coliform, only E. coli, or both were present in samples 1-8.

Samples 1-14 were then investigated for the presence of free indole.

Set of 14 small sterile petri dishes were prepared and 1 ml of solution from vials 1-14 was transferred to these dishes. Filter paper saturated with Kovacs reagent was placed over the petri dish. After 4 hours, the filter was examined for purple red color or spots.

### Results

The filters placed over petri dishes 9-14 were not stained and remained white, indicating the absence of E. coli. Filters 1-8 had a purple red color spots evidencing the presence of E. coli colonies.

### EXAMPLE 4

### "COLITAG" RAPID E.C. TEST - A KIT FOR DETERMINATION OF TOTAL COLIFORM AND E. COLI IN WATER SAMPLES

### SUMMARY

Coliform is a group of Enterobacteriaceae containing both infectious fecal E.coli as well as other noninfectious and environmental bacteria. Only E. coli presence in the water is generally recognized as evidence of sewage contamination.

E. coli is a species of family Enterobacteriaceae. E. coli possess two characteristics: lactose utilization, and indole production distinguishing it from other Enterobacteriaceae and other coliforms.

In detection of total coliform and E. coli, COLITAG RAPID TEST utilizes these two characteristics and determines the presence of β-galactosidase, an enzyme involved in lactose utilization which appear in coliforms including E. coli and tryptophanase, an enzyme involved in indole production, which appear only in fecal E. coli. The medium composition eliminates catabolic repression by lactose substitute and promotes the growth of E. coli. In detection of total coliform, the yellow color derived from β-galactosidase metabolizing ONPG-lactose substitute is observed. In this test, water samples are reacted with COLITAG RAPID TEST medium containing tryptophan and o-nitrophenyl-β-galactopyranoside (ONPG), tryptose, sodium lauryl sulfate, isopropyl-β-D-thiogalactopyranoside and sodium chloride at 35°C for 4 hours and at 44.5°C for 20 hours or until a yellow color develops. The presence of the yellow color indicates a lactose utilization of lactose surrogate ONPG with β-galactosidase. The presence of yellow color indicates the presence of total coliforms including E. coli. Samples containing yellow color are subsequently submitted to indole test, wherein small portion of a yellow colored sampled from step 1 is reacted with Kovacs reagent. Only those samples which give purple-red color contain E. coli. Indole formation evidenced by purple red color thus indicates the presence of E. coli in tested water sample.

### PRODUCT DESCRIPTION, STORAGE

COLITAG RAPID TEST consisting of:

Step 1: 2 sterile glass vials of 20 ml volume with stoppers containing 3 ml of concentrated freeze-dried TAG Medium each.

Concentrated TAG Base Medium contains 3 g of tryptophane, 1.5 g of ONPG, 3 g of isopropyl-β-D-thiogalactopyranoside (ITPG), 9 g of tryptose, and 0.3 g of sodium lauryl sulfate 0.3 g magnesium sulfate, 1.5 g potassium diphosphate, 9 g disodium phosphate, 9 g sodium chloride dissolved in 1000 ml distilled water. Sterilized.

Store the COLITAG RAPID TEST in the refrigerator at 2-8°C until used. Do not open glass vials until ready to use.

Step 2: Spot plate (or microtitre plate) and dropping bottle containing Kovacs reagent.

### SPECIMEN

Water sample to be tested-undiluted.

### TEST PROCEDURE

Principle of Step 1: ONPG substituting for lactose is metabolized with β-galactosidase. At 35°C E. coli grow and utilize ONPG and produce indole, at 44.5°C all other indole producing non E. coli bacteria are killed. β-galactoside turns the medium yellow. In the yellow colored medium, both coliforms and E. coli are present. In samples which do not have a yellow color, there are no coliforms and E. coli.

Principle of Step 2: Tryptophan in the medium provides a nitrogen source for E. coli and is metabolized by E. coli tryptophanase to form free indole, pyruvate and ammonia in equimolar amounts. Since the tryptophan is used as the sole nitrogen source, no other bacteria than E. coli will produce the purple-red color when the Kovacs reagent is added.

### SET-UP

Remove the vials with freeze-dried medium from the refrigerator, open the vial and add the water sample up to 100 ml mark on the vial. Close the vial with the stopper, swirl the vial gently so that the medium and water mix. Incubate at 35°C followed by 44.5°C for 20 hours or until a bright yellow color develops.

Separate vials having no color from those having a yellow color.

With a pasteur pipette add about 1 ml taken from a yellow sample to each well of the spot plate or microtitre plate. Add a drop of Kovacs' reagent and look for the formation of purple-red color within 10 seconds. Record.

### EVALUATION OF RESULTS

### Step 1

Bright yellow color of the samples signify presence of coliform and may or may not include E. coli. No change in color signify absence of coliform and E. coli.

### Step 2

Red-purple color of the sample in spot plate or microtitre plate signifies the presence of free indole and thus the presence of E. coli. Absence of red-purple color signifies the absence of E. coli but presence of coliform.

### EXAMPLE 5

### Multiple Tube Dilution Assay

This example illustrates enumeration method for determination of the most probable number of E. coli present in the water sample.

The method is based on the sequential dilution method generally used in the microbiological testing. Water samples are diluted by several orders to determine the most probable number of E. coli.

This example illustrates the method for quick visual determination of presence or absence and enumeration of E. coli in water samples. The method utilizes two distinct characteristics specific to E. coli., namely utilization of lactose and formation of indole.

The principle of this method is that the lactose which normally inhibits formation of indole, is substituted with substrate which is metabolized or utilized by the same enzyme as lactose, by namely β-galactosidase. In this manner, and due to specifically balanced medium components eliminating the catabolic repression, the substitute for lactose does not inhibit formation of indole from tryptophan.

For this method, a specific tryptophan-galactosidase (TAG) medium number (5), described in Example 3 containing minimal amounts of tryptophan, and lactose substitute ONPG was prepared and used. Medium optionally has isopropyl-β-D-thiogalactopyranoside (IPTG), lactose enzyme production inducer, and tryptose as a secondary nitrogen growth promoter producing compound and is preferably prepared a suitable mineral salts solution. The medium is reacted with normal and diluted samples. From the degree of dilution, the most probable number of E. coli present can be determined.

### Procedure for determination of E. coli

For each sample, the set of five sterile tubes in triplicate was prepared and marked 1A, 1B, 1C, 2A, 2B, 2C, 3A, 3B, 3C, 4A, 4B, 4C and 5A, 5B, 5C; and other description of the sample such as I, II, etc. Each tube has a saline at 10 ml volume.

There were 3 samples for each group
I Controls - containing Salmonella
II E. coli ATCC 25922, ECOR 4, ECOR 5 and ECOR 6
III Coliforms without E. coli, Klebsiella pneumoniae, Citrobacter freundii, Enterobacter cloacae.

Into each of the tubes I 1A - I 5A, II 1A - II 5A, III 1A - III 5A, etc., 10 ml of the TAG media (6) was added up to the line on the tube. Then, 1 ml of sample to be tested was added to tubes 1A, and 1B, 1C to achieve 1/10 dilution. Mixed. 1 ml of solution from tube 1A was removed and transferred to the tube 2A to achieve 1/100 dilution. Mixed. 1 ml of solution from tube 2A was removed and transferred to the tube 3A to achieve 1/1,000 dilution. Mixed. 1 ml of solution 3A was removed and transferred to the tube 4A to achieve dilution 1/10,000. Mixed. 1 ml of solution from the tube 4A was transferred to the 5A to achieve dilution 1/100,000 tubes 1B-5B and 1C-5C were prepared in similar way.

Coliform samples III and E. coli samples II were similarly diluted.

Alternatively, one will make serial dilutions in saline or 0.1% peptone and then put 0.1 or 1.0 ml of each dilution into each of three culture tubes.

Tubes were closed or covered and gently shaken so that water with the medium were properly mixed. The tubes were put in a 35°C incubation for 4 hours, followed by 44.5°C incubation for 20 hours. Following the incubation, samples were visually examined.

### Results:

Control samples of Group I remained colorless, there were no coliforms or E. coli present. E. coli samples of group II were bright yellow, and after adding Kovacs reagent were red-purple. E. coli were present in large number in tubes II 1A, B, C - 3A, B, C. Coliform samples of group III were pale yellow suggesting the presence of coliform. There was slightly visible purple color in samples II 4, A, B, C-5, A, B, C.

This is a COLITAG and E. coli multiple dilution method. The red-purple color indicates that both lactose utilization and indole production characteristic for E. coli are present and thus the presence of E. coli is determined. The number of positive tubes at each dilution, when interpreted with a most-probable number (MPN) table, gives an estimate of the concentrations of coliforms and E. coli in the original samples.

### EXAMPLE 6

### ECOTAG TEST - A KIT FOR QUANTITATION OF E. COLI IN WATER SAMPLES

### SUMMARY

E. coli presence in the water is generally recognized as evidence of sewage contamination. Under the present regulations issued by EPA, the current permissible level of contamination of drinking water is around 5% of total coliform. If the fecal E. coli is present, it shows up within the coliform group. However, not all coliforms are of fecal origin and thus it is necessary to determine approximate most probable number of E. coli present within the allowable 5% of total coliform.

E. coli is a species of family Enterobacteriaceae and is one of the coliforms. E. coli possess two characteristics: lactose utilization, and indole production distinguishing it from other Enterobacteriaceae and other coliforms.

In detection of most probable number of E. coli, ECOTAG TEST utilizes these two characteristics and determines the presence the yellow or red-purple color in 10 fold diluted sequence of samples. In detection of E. coli presence, water samples are sequentially diluted and reacted with ECOTAG QUANTI TEST medium containing essentially tryptophan and o-nitrophenyl-β-galactopyranoside (ONPG) and reaction enhancers at 35°C for 4 hours and 44.5°C for 20 hours or until a yellow color develops. The presence of the yellow color indicates the presence of coliform and/or E. coli in tested water sample. After reaction with Kovacs reagents, the red-purple color develops in samples containing E. coli. The number of E. coli depends on the dilution.

### PRODUCT DESCRIPTION, STORAGE

ECOTAG QUANTI TEST consisting of:

For one sample:

15 sterile tubes with stoppers having a line at 10 ml level containing originally 10 ml of concentrated freeze-dried TAG Medium Base each. Marked x for sample identification and subdivided into 1A-5A, 1B-5B, 1C-5C.

Concentrated TAG Base Medium contains 3 g of tryptophane, 1.5 g of ONPG, 3 g of isopropyl-β-D-thiogalactopyranoside, 0.3 g of sodium lauryl sulfate dissolved in in mineral salts at 3 times the usual concentration. Sterilized.

Dropping vial with Kovacs reagent.

Store the CHANG QUANTI E.C. TEST in the refrigerator at 2-8°C until used. Do not open glass tubes until ready to use.

### SPECIMEN

Water sample to be tested-undiluted.

### TEST PROCEDURE

Principle: Tryptophan in the medium provides a nitrogen source for E. Coli and is metabolized by E. coli tryptophanase to form free indole, pyruvate and ammonia in equimolar amounts. ONPG substituting for lactose is metabolized with β-galactosidase. At 35°C E. coli grow and utilize ONPG and produce indole and at 44.5°C all other indole producing non E. coli bacteria are killed. The action of β-galactosidase on ONPG turns the medium yellow. Since the tryptophan is used as the sole nitrogen source, no other bacteria than coliform or E. coli will produce the yellow color under these conditions. E. coli will turn red-purple with Kovacs reagent.

### SET-UP

Remove the tubes with freeze-dried medium from the refrigerator, open the tube and add the sterile distilled water up to 10 ml mark on the vial.

Pipette 1 ml of water sample to be tested into tubes x 1A, x 1B and x 1C. Mix.

Remove 1 ml of sample from the tube x 1A and transfer to the tube x 2A. Mix.

Remove 1 ml of sample from the tube x 2A and transfer to the tube x 3A. Mix.

Remove 1 ml of sample from the tube x 3A and transfer to the tube x 4A. Mix.

Remove 1 ml of sample from the tube x 4A and transfer to the tube x 5A. Mix.

In the same manner, prepare samples x 1B - x 5B and x 1C - x 5C.

Incubate at 35°C for 4 hours, then at 44.5°C for 20 hours. Record the presence or absence of yellow color.

Close the vial with the stopper, swirl the vial thoroughly so that the medium and water mix. Incubate at 35°C and 44.5°C until a bright yellow color develops or 24 hours whatever is shorter. Add 2-3 drops of Kovacs reagent.

### EVALUATION OF RESULTS

Bright yellow color of the solution signify presence of coliform and/or E. coli. Red-purple color signifies presence of E. coli. No change in color signify absence of or an undeterminably small amount of E. coli.

Dilution:
Samples x 1 A, B, C = 1/10
Samples x 2 A, B, C = 1/100
Samples x 3 A, B, C = 1/1,000
Samples x 4 A, B, C = 1/10,000
Samples x 5 A, B, C = 1,100,000
Samples are evaluated for colony forming units and these are recorded and expressed per dilution.

### EXAMPLE 7

### ECOTAG AND COLITAG Membrane Filter Techniques

This example illustrates another methodological variation for ECOTAG and COLITAG tests.

### Materials:

Sterile membrane filters. Examples, recommended in various Standard Methods publications, include 47 mm, 0.45 µm plain or gridded membranes such as Gelman GNG. Hydrophobic grid membrane filters (HMGF) can also be used.

### Filter Apparatus:

A suitable sterilizable or pre-sterilized disposable filter funnel and flask. Also a manual or electrical device for drawing a water sample through the membrane filter.

### Medium:

Liquid medium of the COLITAG or ECOTAG type kept from flowing by incorporation into a gel (typically a 1.5% agar gel) or by saturation of a sterile filter paper pad (typically 2 ml of medium on a 55 mm diameter pad). The medium is pre-sterilized and held in a suitable sterile container such as a Petri dish.

### Typical Use:

Carefully filter a 100 ml water sample through the membrane filter, using the filter apparatus. Then remove the filter with sterile forceps and place it carefully onto a pad saturated with 2 ml of COLITAG-X-GAL medium. COLITAG-X-GAL is identical to the COLITAG medium described earlier, except that 0.1 g X-GAL is used in place of 0.5 g ONPG per 1000 ml of medium.

Incubate the filter, pad, and medium inside the protecting Petri dish at 35°C for 4 hours, then at 44.5°C for 20 hours. Then inspect the colonies and make a note of the ones having blue color. These are coliforms.

To determine which coliform colonies are E. coli, transfer the membrane filter to a filter paper pad saturated with Kovacs' reagent.

After two minutes, look for a reddish tint, indicating a colony of indole-positive bacteria. Indole-positive coliforms are probably E. coli. It may be easier to see the reddish color by gently lifting the filter and inspecting the bottom side.

### Data Collection:

The number of blue colonies is the coliform count in colony-forming units (cfu) per 100 ml. The number of blue colonies which turn reddish upon contact with Kovacs' (or Ehrlich's) reagent is the E. coli count in cfu per 100 ml.

### EXAMPLE 8

### Comparison of Colitag Test with the Standard Fecal Coliform Test

This example illustrates advantages of the current TAG media when compared to the Standard Fecal Coliform test using EC medium used to test surface and groundwater samples.

### Procedure

Methodology used for testing samples using E. coli medium, including the description of ingredients is described in Standard Methods, APHA (1985). Methodology used for testing Colitag is according to the method described in Example 1. Water samples were either obtained from surface water or from groundwater. Water was sampled in a sterile 2 liter bottle and brought promptly to the laboratory. Within minutes after arrival in the laboratory, it was diluted in the following way.

### Sample

1. 1 liter water sample;
2. 10 ml of sample (1) was added to test tube containing 10 m of double strength EC or Colitag medium;
3. 1 ml of sample (1) was added to test tube containing 10 m of single strenght EC or Colitag medium;
4. 0.1 ml of sample (1) was added to test tube containing 10 m of single strenght EC or Colitag medium;
5. Dilute sample (1) to, 10:1 solution (5) and add 0.1 of diluted solution (5) to test tube containing 10 m of single strenght EC or Colitag medium;
6. Dilute solution (5) ten-fold and add 0.1 ml to each tube containing 10 m of single strenght EC or Colitag medium.

Fecal coliform test conditions were according to Standard Method. Colitag tubes were incubated for 4 hours at 35°C and for 20 hours at 44.5°C. Then the tubes were examined for ONPG, MUG and indole reactions. The number of positive reactions among tubes was used in conjunction with standard "Most Probable Number" (MPN) table to obtain MPN for the positive E. coli organisms in the original sample.

**TABLE 1**

| Comparision of COLITAG with the Standard Fecal Coliform Test on Surface and Groundwater | | | | |
|---|---|---|---|---|
| **Sample #** | **EC Medium Fecal Coliform** | **Colitag Medium** | | |
| | | **ONPG** | **MUG** | **INDOLE** |
| Suface Water | | | | |
| 3 | 500 | 300 | 170 | 300 |
| 4 | 2800 | 5000 | 5000 | 500 |
| 7 | 1300 | 3000 | 3000 | 3000 |
| 10 | 800 | 800 | 800 | 800 |
| 12 | 110 | 220 | 220 | 800 |
| 13 | 300 | 300 | 300 | 300 |
| | | | | |
| Ground Water | | | | |
| 8 | <2 | <2 | <2 | <2 |
| 9 | 5000 | 24,000 | 2700 | 24,000 |

Results are illustrated in Table 1. In the surface water, samples 3, 4, 7, 10, 12, and 13 show substantial differences between EC medium which measures total coliform including E. coli, and Colitag test measuring ONPG, MUG and indole. With exception of Sample 3, all results were higher in Colitag test. Moreover, the Colitag test was able to distinguish between MUG-positive and MUG-negative samples, as seen in Sample (3) where the MUG MPN was lower than MPN ONPG and indole. The EC test resulted in MPN 500 probably because coliforms were present in addition to E. coli. In all other samples, Colitag test found large MPN of E. coli than Fecal Coliform test.

In ground water sample (9), again Fecal Coliform showed lower MPN than ONPG and indole of Colitag. MUG MPN again was significantly (p<0.05) lower than MPN for ONPG or indole. Thus, MUG negative E. coli were found to be present. MPN for ONPG and indole are around 5 times higher than MPN of Fecal Coliform and almost 10 times higher than MUG MPN.

### EXAMPLE 9

### The Effect of Temperature on E. coli Test

This example illustrates different results and false positive results obtained at 35°C and elimination of false positive results by introduction of 44.5°C.

### Procedure

Samples were obtained from surface water as in Example 8. Medium composition is shown in the Table 2. Three methods, namely lauryl tryptose EC medium - Standard Fecal Coliform, Colilert (MMO-MUG) described in Fed. Reg., 54 (135): 29999 (July 1989) and Colitag were used for comparison.

Sampling, dilution and tests are as described in Example 8, or in Examples 1-6.

**TABLE 2**

| Comparison of COLITAG (35°C) and COLILERT (35°C) | | | | |
|---|---|---|---|---|
| **Medium** | **Fecal Coliform** | **ONPG** | **MUG** | **Indole** |
| Lauryl tryptose and EC broth (Standard Method) | 80 | - | - | - |
| COLITAG | | 2400 | 1300 | 5000 |
| COLILERT | | 1300 | 300 | |

Sample = Surface water. Data are 5 tube MPN (#/100 ml)

As seen from Table 2, the data in the MUG column clearly show the superiority of Colitag test ONPG MPN was 2400, MUG MPN was 1300 and indole MPN was 5000 over Colilert test where ONPG MPN was only 1300 and MUG MPN only 300.

Thus, Colitag is much more sensitive than Colilert. The difference in sensitivity of MUG tests is significant at the 95% level.

Conclusion of this experiment was to seek another temperature at which the false positive values obtained for indole would not occur.

Table 3 illustrates the results obtained in experiments where the incubation was carried out for 4 hours at 35°C and for further 20 hours at 44.5°C. These incubation conditions solved the problem of false positive results as seen in Table 3 at 35°C, where not only E. coli but also other bacteria, such as Ent. agglomerans, Ser. Marcescans, K. oxytoca and others, which are indole positive, gave indole positive results, thus suggesting the presence of E. coli where there was none.

**TABLE 3**

| SAMPLE | STANDARD METHODS FECAL COLIFORM MPN (#/100 ML) | 35°C COLITAG | | 44.5°C COLITAG | |
|---|---|---|---|---|---|
| | | INDOLE MPN (#/100ML) | INDOLE POSITIVE BACTERIA | INDOLE MPN (#/100ML) | INDOLE POSITIVE BACTERIA |
| 3 (Creek) | 500 | 300 | (not done) | 300 | E. coli |
| 4 (Creek) | 2800 | 30,000 | E. coli Ent. agglomerans Ser. Marcescans | 500 | E. coli |
| 7 (puddle in lawn) | 1300 | >160,000 | E. coli K. oxytoca | 3000 | E. coli |
| 10 (Creek) | 800 | 90,000 | E. coli Ent. agglomerans P. stuarti | 800 | E. coli |
| 12 (Lake) | 110 | 3000 | E. coli Ent. agglomerans | 800 | E. coli |
| 13 (Lake) | 300 | 13,000 | (not done yet) | 300 | (not done yet) |
| 9 (Well) | 5000 | 11,000 | E. coli C. freundii | 24,000 | E. coli |

Incubation at 44.5°C solves the problem of false - positive indole tests. At 44.5°C, only E. coli was found in indole-positive Colitag tubes.

When these same samples were incubated at 35°C for only 2-4 hours and the rest of the time at 44.5°C, only E. coli were found to be present as seen in 44.5°C Colitag Test column. The identity of E. coli was confirmed by isolation on EMB agar plates and sole E. coli presence was confirmed with Interotube-2 test or with API-20E. In these isolates, there were no other indole positive bacteria.

## Claims

1. A method for specifically detecting coliform bacteria and *E.coli* in a water sample, which method comprises steps:
(a) contacting said sample with a medium comprising:
(i) a lactose surrogate which is a β-D-galactosidase substrate;
(ii) tryptophan or a tryptophan-substitute which is a tryptophanase substrate;
(ii) a protein or peptide hydrolysate in an amount sufficient to promote the growth of *E.coli;*
(b) incubating said bacteria at a temperature of 44.5°C for 2 to 24 hours; then
(c) determining the presence or absence of a β-D-galactosidase reaction product of said β-D-galactosidase substrate;
(d) determining the presence or absence of a tryptophanase reaction product of said tryptophanase substrate;
wherein the presence of said β-D-galactosidase reaction product indicates the presence of a coliform bacterium in said sample and the presence of said tryptophanase reaction product indicates the presence of *E.coli* in said sample.

2. A method for specifically detecting coliform bacteria and *E.coli* in a water sample, which method comprises steps:
(a) forming an aqueous sample medium mixture by contacting said sample with a medium comprising:
(i) an indole-producing tryptophanase substrate;
(ii) a chromogenic or fluorogenic β-D-galactosidase substrate;
(iii) a protein or peptide hydrolysate in an amount sufficient to promote the growth of *E.coli* in said mixture;
(b) incubating said mixture at a temperature of 44.5°C for 2 to 24 hours; thereafter
(c) determining the presence or absence of a first color or a first fluorescence in said mixture;
(d) adding an indole detection reagent to said mixture; thereafter
(e) determining the presence or absence of a second color in said mixture;
wherein the presence of said first color or said first fluorescence in said mixture indicates the presence of a coliform bacterium in said sample and the presence of said second color in said mixture indicates the presence of *E.coli* in said sample.

3. A method for specifically detecting coliform bacteria and *E.coli* in a water sample, which method comprises steps:
(a) forming an aqueous sample-medium mixture by contacting said sample with a medium comprising:
(i) tryptophan;
(ii) ortho-nitrophenyl-β-D-galactopyranoside;
(iii) a protein or peptide hydrolysate in an amount sufficient to promote the growth of *E.coli;*
(b) incubating said mixture at a temperature of 44.5°C for 2 to 24 hours; thereafter
(c) determining the presence or absence of a yellow color in said mixture;
(d) adding p-dimethylaminobenzaldehyde in acidified n-butyl alcohol or acidified isoamylalcohol to said mixture; thereafter
(e) determining the presence or absence of a red-purple color in said mixture;
wherein the presence of said yellow color in said mixture indicates the presence of a coliform bacterium in said sample and the presence of said red-purple color in said mixture indicates the presence of *E.coli* in said sample.

4. A method according to claim 1 or 2 wherein said β-D-galactosidase substrate is ortho-nitrophenyl-β-D-galactopyranoside.

5. A method according to claim 1 or 2 wherein said tryptophanase substrate is tryptophan.

6. A method according to claim 2 wherein said indole detection reagent is p-dimethylaminobenzaldehyde in acidified n-butyl alcohol or acidified isoamylalcohol.

7. A method according to claim 1, 2 or 3 wherein said hydrolysate is trypytose.

8. A method according to claim 1, 2 or 3 wherein said hydrolysate is present at a concentration from 0.05 to 30 g/l.

9. A method according to claim 1, 2 or 3 wherein said medium further comprises an inhibitor of bacterial growth selected from the group consisting of a detergent, an antibiotic and a bile salt.

10. A method according to claim 1, 2 or 3 further comprising filtering said sample through a filter prior to said contacting step.

## Patentansprüche

1. Verfahren zum spezifischen Nachweis von koliformen Bakterien und E.coli in einer Wasserprobe, wobei das Verfahren die folgenden Stufen umfaßt:
(a) Zusammenbringen der Probe mit einem Medium, umfassend
(i) ein Laktosesurrogat, das ein β-D-Galaktosidasesubstrat ist,
(ii) Tryptophan oder einen Tryptophanersatz, das/der ein Tryptophanasesubstrat ist,
(iii) ein Protein- oder Peptidhydrolysat in einer ausreichenden Menge, um das Wachstum von E.coli zu beschleunigen,
(b) Inkubieren der Bakterien bei einer Temperatur von 44,5°C während 2 bis 24 Stunden, anschließend
(c) Bestimmung des Vorhandenseins oder Nichtvorhandenseins eines β-D-Galaktosidasereaktionsproduktes des β-D-Galaktosidasesubstrats,
(d) Bestimmung des Vorhandenseins oder Nichtvorhandenseins eines Tryptophanasereaktionsproduktes des Tryptophanasesubstrats,
wobei das Vorhandensein des β-D-Galaktosidasereaktionsproduktes das Vorhandenseins eines koliformen Bakteriums in der Probe und das Vorhandenseins des Tryptophanasereaktionsproduktes das Vorhandensein von E.coli in der Probe anzeigt.

2. Verfahren zum spezifischen Nachweis von koliformen Bakterien und E.coli in einer Wasserprobe, wobei das Verfahren die folgenden Stufen umfaßt:
(a) Bildung eines Gemisches eines wäßrigen Probenmediums durch Zusammenbringen der Probe mit einem Medium umfassend
(i) ein Indol-bildendes Tryptophanasesubstrat,
(ii) ein chromogenes oder fluorogenes β-D-Galaktosidasesubstrat,
(iii) ein Protein- oder Peptidhydrolysat in einer ausreichenden Menge, um das Wachstum von E.coli in dem Gemisch zu beschleunigen,
(b) Inkubieren des Gemisches bei einer Temperatur von 44,5°C während 2 bis 24 Stunden, anschließend
(c) Bestimmung des Vorhandenseins oder Nichtvorhandenseins einer ersten Färbung oder einer ersten Fluoreszenz in dem Gemisch,
(d) Zugabe eines Indolnachweisreagenz zu dem Gemisch und anschließend
(e) Bestimmung des Vorhandenseins oder Nichtvorhandenseins einer zweiten Färbung in dem Gemisch, wobei das Vorhandensein der ersten Färbung oder der ersten Fluoreszenz in dem Gemisch das Vorhandensein eines koliformen Bakteriums in der Probe und das Vorhandensein der zweiten Färbung in dem Gemisch das Vorhandensein von E.coli in der Probe anzeigt.

3. Verfahren zum spezifischen Nachweis von koliformen Bakterien und E.coli in einer Wasserprobe, wobei das Verfahren die folgenden Stufen umfaßt:
(a) Bildung eines Gemischs des wäßrigen Probenmediums durch Zusammenbringen der Probe mit einem Medium umfassend
(i) Tryptophan,
(ii) o-Nitrophenyl-β-galaktopyranosid,
(iii) ein Protein- oder Peptidhydrolysat in einer ausreichenden Menge, um das Wachstum von E.coli zu beschleunigen,
(b) Inkubieren des Gemisches bei einer Temperatur von 44,5°C während 2 bis 24 Stunden, anschließend
(c) Bestimmung des Vorhandenseins oder Nichtvorhandenseins einer gelben Färbung in dem Gemisch,
(d) Zugabe von p-Dimethylaminobenzaldehyd in angesäuertem n-Butylalkohol oder angesäuertem Isoamylalkohol zu dem Gemisch und anschließend
(e) Bestimmung des Vorhandenseins oder Nichtvorhandenseins einer rot-purpurnen Färbung in dem Gemisch,
wobei das Vorhandensein der gelben Farbe in dem Gemisch das Vorhandensein eines koliformen Bakteriums in der Probe und das Vorhandensein der rot-purpurnen Färbung in dem Gemisch das Vorhandensein von E.coli in der Probe anzeigt.

4. Verfahren nach Anspruch 1 oder 2, wobei das β-D-Galaktosidasesubstrat o-Nitrophenyl-β-D-galaktopyranosid ist.

5. Verfahren nach Anspruch 1 oder 2, wobei das Tryptophanasesubstrat Tryptophan ist.

6. Verfahren nach Anspruch 2, wobei das Indolnachweisreagenz p-Dimethylaminobenzaldehyd in angesäuertem n-Butylalkohol oder angesäuertem Isoamylalkohol ist.

7. Verfahren nach Anspruch 1, 2 oder 3, wobei das Hydrolysat Tryptose ist.

8. Verfahren nach Anspruch 1, 2 oder 3, wobei das Hydrolysat in einer Konzentration von 0,05 bis 30 g/l vorhanden ist.

9. Verfahren nach Anspruch 1, 2 oder 3, wobei das Medium zusätzlich einen Inhibitor für das Bakterienwachstum enthält, ausgewählt aus der Gruppe bestehend aus einem Detergenz, einem Antibiotikum und einem Gallensäuresalz.

10. Verfahren nach Anspruch 1, 2 oder 3, umfassend ferner das Filtrieren der Probe durch ein Filter vor der Stufe des Zusammenbringens.

## Revendications

1. Procédé permettant de détecter spécifiquement des bactéries coliformes et *E. coli* dans un échantillon d'eau, lequel procédé comprend les étapes consistant
(a) à mettre en contact ledit échantillon avec un milieu contenant
(i) un substitut de lactose qui est un substrat de la β-D-galactosidase,
(ii) du tryptophane ou un substitut de tryptophane qui est un substrat de la tryptophanase,
(iii) un produit d'hydrolyse protéique ou peptidique en une quantité suffisante pour favoriser la croissance *d'E. coli;*
(b) à incuber lesdites bactéries à une température de 44,5 °C pendant 2 à 24 heures, puis
(c) à déterminer la présence ou l'absence d'un produit résultant de l'action de la β-D-galactosidase sur ledit substrat de la β-D-galactosidase,
(d) à déterminer la présence ou l'absence d'un produit résultant de l'action de la tryptophanase sur ledit substrat de la tryptophanase, procédé dans lequel la présence dudit produit résultant de l'action de la β-D-galactosidase indique la présence d'une bactérie coliforme dans ledit échantillon et la présence dudit produit résultant de l'action de la tryptophanase indique la présence d'*E. coli* dans ledit échantillon.

2. Procédé permettant de détecter spécifiquement la présence de bactéries coliformes et d'*E. coli* dans un échantillon d'eau, lequel procédé comprend les étapes consistant
(a) à préparer un mélange échantillon aqueux/milieu en mettant en contact ledit échantillon avec un milieu contenant :
(i) un substrat de la tryptophanase donnant de l'indole,
(ii) un substrat chromogène ou fluorogène de la β-D-galactosidase,
(iii) un produit d'hydrolyse protéique ou peptidique en une quantité suffisante pour favoriser la croissance d'*E. coli* dans ledit mélange,
(b) à incuber ledit mélange à une température de 44,5 °C pendant 2 à 24 heures, puis
(c) à déterminer la présence ou l'absence d'une première couleur ou d'une première fluorescence dans ledit milieu,
(d) à ajouter audit mélange un réactif permettant la détection d'indole, puis
(e) à déterminer la présence ou l'absence d'une seconde couleur dans ledit mélange,
procédé dans lequel la présence de ladite première couleur ou de ladite première fluorescence dans ledit mélange indique la présence d'une bactérie coliforme dans ledit échantillon et la présence de ladite seconde couleur dans ledit mélange indique la présence d'*E. coli* dans ledit échantillon.

3. Procédé permettant de détecter spécifiquement la présence de bactéries coliformes et d'*E. coli* dans un échantillon d'eau, lequel procédé comprend les étapes consistant
(a) à préparer un mélange échantillon aqueux/milieu en mettant en contact ledit échantillon avec un milieu contenant :
(i) du tryptophane,
(ii) de l'ortho-nitrophényl-β-D-galactopyranoside,
(iii) un produit d'hydrolyse protéique ou peptidique en une quantité suffisante pour favoriser la croissance *d'E. coli,*
(b) à incuber ledit mélange à une température de 44,5 °C pendant 2 à 24 heures, puis
(c) à déterminer la présence ou l'absence d'une couleur jaune dans ledit mélange,
(d) à ajouter audit mélange du p-diméthylaminobenzaldéhyde dans du n-butanol acidifié ou dans de l'alcool isoamylique acidifié, puis
(e) à déterminer la présence ou l'absence d'une couleur pourpre dans ledit mélange,
procédé dans lequel la présence de ladite couleur jaune dans ledit mélange indique la présence d'une bactérie coliforme dans ledit échantillon et la présence de ladite couleur pourpre dans ledit mélange indique la présence *d'E. coli* dans ledit échantillon.

4. Procédé conforme à la revendication 1 ou 2 dans lequel ledit substrat de la β-D-galactosidase est l'ortho-nitrophényl-β-D-galactopyranoside.

5. Procédé conforme à la revendication 1 ou 2 dans lequel ledit substrat de la tryptophanase est le tryptophane.

6. Procédé conforme à la revendication 2 dans lequel ledit réactif permettant la détection d'indole est le p-diméthylaminobenzaldéhyde dans de l'alcool n-butylique acidifié ou dans de l'alcool isoamylique acidifié.

7. Procédé conforme à la revendication 1, 2 ou 3 dans lequel ledit produit d'hydrolyse est une tryptose.

8. Procédé conforme à la revendication 1, 2 ou 3 dans lequel ledit produit d'hydrolyse est présent à raison de 0,05 à 30 g/l.

9. Procédé conforme à la revendication 1, 2 ou 3 dans lequel ledit milieu contient en outre un inhibiteur de la croissance bactérienne choisi dans le groupe formé par les détergents, les antibiotiques et les sels biliaires.

10. Procédé conforme à la revendication 1, 2 ou 3 consistant en outre à filtrer ledit échantillon sur un filtre avant l'étape de mise en contact.
